# EUROPEAN PATENT SPECIFICATION

(11) **EP 1 968 620 B1**
(45) Date of publication and mention of the grant of the patent: **18.04.2012**
(21) Application number: 06839082.2
(22) Date of filing: 06.12.2006
(51) Int. Cl.: A61K 36/8962, A61K 36/00, A61P 31/00

(54) **Compositions comprising powdered garlic (Allium sativum linn.) and a concentrate of organic selenium compounds for nutritional supplementation**
Zusammensetzungen die Knoblauchpulver (Allium sativum Linn.) und ein Konzentrat von organischen Selenverbindungen enhalten zur Nahrungsergänzung
Compositions comprenant de l'ail en poudre (Allium sativum linn.) et un concentré de composés organiques de selenium pour la supplementation nutritionnelle

(30) Priority: 06.12.2005 US 164787
(43) Date of publication of application: 17.09.2008
(73) Proprietor: Majeed, Muhammed, Piscataway, NJ 08854 (US); Bammi, Rajinder Kumar, Bangalore 560058 (IN); Badmaev, Vladimir, Piscataway, NJ 08854 (US); Prakash, Subbalakshmi, Piscataway, NJ 08854 (US); Nagabhushanam, Kalyanam, Piscataway, NJ 08854 (US)
(72) Inventor: Majeed, Muhammed, Piscataway, NJ 08854 (US); Bammi, Rajinder Kumar, Bangalore 560058 (IN); Badmaev, Vladimir, Piscataway, NJ 08854 (US); Prakash, Subbalakshmi, Piscataway, NJ 08854 (US); Nagabhushanam, Kalyanam, Piscataway, NJ 08854 (US)
(74) Representative: Heinze, Ekkehard
(86) International application number: PCT/US2006/046519
(87) International publication number: WO 2007/067600

(56) References cited:
- WO-A1-00/33860
- US-B1- 6 794 537
- IP CLEMENT ET AL: "Chemical speciation influences comparative activity of selenium-enriched garlic and yeast in mammary cancer prevention" JOURNAL OF AGRICULTURAL AND FOOD CHEMISTRY, vol. 48, no. 6, June 2000 (2000-06), pages 2062-2070, XP002591368 ISSN: 0021-8561
- CLEMENT IP ET AL: "CHEMOPREVENTION OF MAMMARY CANCER WITH SE-ALLYLSELENOCYSTEINE AND OTHER SELENOAMINO ACIDS IN THE RAT" ANTICANCER RESEARCH, INTERNATIONAL INSTITUTE OF ANTICANCER RESEARCH, GR, vol. 19, no. 4B, 1 January 1999 (1999-01-01), pages 2875-2880, XP008041664 ISSN: 0250-7005
- BIRD S M ET AL: "High-performance liquid chromatography of selenoamino acids and organo selenium compounds Speciation by inductively coupled plasma mass spectrometry" JOURNAL OF CHROMATOGRAPHY, ELSEVIER SCIENCE PUBLISHERS B.V, NL LNKD- DOI:10.1016/S0021-9673(97)00657-2, vol. 789, no. 1-2, 21 November 1997 (1997-11-21), pages 349-359, XP004099958 ISSN: 0021-9673
- MILNER J.: 'Garlic: Its Anticarcinogenic and Antitumorigenic Properties' NUTRITION REVIEWS vol. 54, no. 11, November 1996, pages 82 - 86, XP008084343
- BLOCK E. ET AL.: 'Allium Chemistry Synthesis, Natural Occurence, Biological Activity and Chemistry of Se-alk(en)ylselenocysteines and their g-glutamyl derivatives and oxidation products' AGRIC. FOOD CHEM. vol. 49, 2001, pages 458 - 470, XP002365711
- KOTREBAI M. ET AL.: 'High-Performance Liquid Chromatography of Selenium Compounds Utilizing Perfluorinated Carboxylic Acid Ion-Pairing Agents and Inductively Coupled Plasma and Electrospray Ionization Mass Spectrometric Detection' JOURNAL OF CHROMATOGRAPHY A vol. 866, 2000, pages 51 - 63, XP003015901
- HONGHONG ET AL.: 'Identification of Selenium Species in Selenium-Enriched Garlic, Onion and Broccoli Using High-Performance Ion Chromatography With Inductively Coupled Plasma Mass Spectrometry Detection' ANALYTICAL COMMUNICATIONS vol. 33, August 1996, pages 279 - 281, XP008084342

## Description

### Technical field

The present invention is related to compositions of garlic and a concentrate of organic selenium compounds, and the use of these compositions for nutritional supplementation in humans and animals.

### Background art

Selenium is a vital trace element nutrient with multiple roles in the growth and functioning of living cells in higher animals and humans. At the molecular level, selenium (as selenocysteine) is an essential component of the active sites of the antioxidant enzyme glutathione peroxidase, and the enzymes participating in thyroid functions iodothyronine-5'-deiodinase and mammalian thioredoxin reductase. Selenium is also present in several other mammalian selenoproteins. Low selenium status has been linked with the occurrence of decreased immunity to diseases and the prevalence of various forms of cancer.

Selenium occurs in foods, through uptake by plants from the soil, in the form of the selenoamino acids, selenomethionine and selenocysteine, and their derivatives. However, as the element is unevenly distributed in the earth's crust, dietary supplementation is often needed. Organic selenium compounds are preferred for supplementation on account of their superior bioavailability. An ideal nutritional supplement would be a selenium enriched edible plant part wherein the selenium metabolically accumulates in the form of bioavailable organic selenium compounds. Plants that naturally contain higher levels of the sulfur containing amino acids such as those from the Allium and Brassica species are preferred for enrichment, based on metabolic criteria. Selenium-enriched garlic is reported to be useful as a nutritional supplement in the prevention of cancer (Ip, et al., 1992, 1996; Ip and Lisk, 1993, 1997; Lu et al., 1996).

Clinical intervention trials and in vitro data revealed the efficacy of selenium in the form of selenium yeast or selenomethionine in cancer prevention (Clark et al., 1996, 1998). Ip et al (2000) described the role of chemical speciation on the comparative activity of selenium-enriched garlic and selenium yeast in mammary cancer prevention in rats, wherein selenium-enriched garlic was shown to be more efficacious than selenium yeast. γ-glutamyl Se-methylselenocysteine is reported to be the major form of selenium in selenium-enriched garlic while L-Selenomethionine was shown to be the major form of selenium in selenium enriched yeast. However, the present inventors have for the first time identified Allylselenocysteine as yet another major selenoamino acid present in selenium enriched garlic bulbs.

Laboratory studies indicate that γ-Glutamyl-Se-methyl-L-selenosysteine is an effective chemopreventive agent, serving as a carrier for Se-Methyl-L-selenocysteine (Dong et al., 2001; Medina et al. , 2001). Se-Methyl-L-selenocysteine is a well researched chemopreventive organoselenium compound, which is not incorporated in the body proteins, and is therefore less toxic than other forms of supplemental selenium (Ip et al., 1994; Medina et al., 2001).

US Patent application No. 10/249,239 disclosed a novel process for preparing garlic bulbs naturally enriched with an unique composition of organic selenium compounds through a soilless culture technique. US Patent application No. 10/605,578 described a process for preparing a concentrate containing organic selenium compounds extracted from enriched garlic bulbs of US Patent application No. 10/249,239, for nutritional supplementation in humans and animals.

### Disclosure of the Invention

The invention discloses compositions comprising 10 - 500 ppm of Methylselenic acid, Allyl Selenocysteine, the Selenoamino acids L-Selenomethionine and Se-Methyl-L-selenocysteine; and Selenoamino acid dipeptides, N-γ (L-Glutamyl) L-selenomethionine, and N-γ (L-Glutamyl) Se-methyl-L-selenocysteine, A dispersion of these compounds is made in natural garlic powder containing 10 to 500 ppm of Alliin, for nutritional supplementation.

### Best mode for carrying out the invention

Example 1: Process to prepare a composition containing selectively fractionated bioactive organic selenium compounds from selenium-enriched Allium sativum Linn bulbs:

100 kg of selenium-enriched garlic bulbs prepared by soilless culture technique were crushed and subjected to multi-stage supercritical fluid extraction followed by chromatographic separation. High pressure carbon dioxide (10 to 60 MPa), modified with ethanol and water (50:50), was used to extract Selenium containing non-protein amino acids as well as selenoamino acid dipeptides. These were separated and purified using preparative HPLC, the mobile phase and water were removed by evaporation under reduced pressure and freeze drying, to yield bioactive selenoamino acid and selenoamino acid dipeptide fractions. The fractions obtained were blended with natural garlic powder to yield a composition containing 100 to 2000 ppm of selenium in the form of organic selenium compounds.

An exemplary composition of the selenium enrichment concentrate configured to provide 1000 ppm organic selenium content in natural garlic powder containing 200 ppm Alliin is detailed in Table 1.

Table 1: Composition of the Enrichment Concentrate

**Table 1**

| Component | Level in ppm | Elemental selenium equivalent (ppm) |
|---|---|---|
| Methylselenic Acid | 25 | 15.6 |
| Allylselenocysteine | 378 | 143.6 |
| Se-methyl-L-selenocysteine | 120 | 52.3 |
| L-selenomethionine | 75 | 30.9 |
| N-γ (L-Glutamyl) L-selenomethionine | 60 | 15.0 |
| N-γ (L-Glutamyl) Se-methyl-L-selenocysteine | 344 | 87.0 |
| | Total =1002 | 344.4 |

Example 2: Toxicological studies on the selenium enriched garlic powder

The study was designed to determine the acute oral toxicity of garlic powder enriched with the mixture of Example 1, to Sprague Dawley rats (male and female) of age 6 to 8 weeks with weight ranging from 124.1 g to 140.6 g. The rats were randomly selected in groups of five of like sex.

The test substance suspended in water was administered by oral route to rats, at a dose volume of 10ml/kg. Ten rats (5 male and 5 female) were allocated to a treatment of 2000 mg/kg of the test composition. The rats were observed for 14 days after treatment. All animals survived through the study period of 14 days and were free of intoxicating signs 1 to 2 hours after the treatment. The LD50 value of the composition in rats by oral route was found to be greater than 2000 mg/kg body weight.

### Example 3: Clinical efficacy and safety of the composition

Study Objective: To determine the efficacy and safety of the composition in reducing oxidative stress levels.

Study Design: 42-day open prospective single centre study to measure the efficacy and safety of composition. In this 42 day study, each patient was administered the composition (providing 100 mcg of elemental selenium) in a single dose, daily after a meal. Selection of Subjects: The selection criteria stipulated were that the subjects have a BMI (body mass index) of 20-38 kg/m2, fall in the age group of 35 to 60 years, with an increased risk of heart disease. These subjects would be otherwise in good health, with no history of alcohol or drug abuse, and no known allergies. Kidney and liver functions would be normal. 13 subjects (in the age group of 25 to 43 years (6 males and 7 females)), participated in the study.

Study Procedures: Efficacy was assessed based on change in oxidative stress, as evidenced by measuring antioxidant profile. Safety was assessed based on standard liver function and kidney function tests. Adverse events, if any, were recorded. Liver function is assessed by using specific enzyme markers. For example, both SGPT (Serum Glutamate-Pyruvate Transaminase) and SGOT (Serum Glutamate-Oxaloacetate Transaminase) are important in the clinical diagnosis of disease. These enzymes, particularly abundant in heart and in liver, are released from injured cells in myocardial infarction, infections, liver damage or cardiovascular disease. Kidney function tests seek to evaluate how well the kidneys filter and transport metabolic waste from the blood into the urine. Serum creatinine, serum urea, and electrolyte (sodium, potassium and chloride) levels are measured. ESR (Erythrocyte sedimentation Rate) and haemoglobin levels, if abnormal, provide an indication of the presence of disease.

Results: Efficacy: From day 0 (baseline) to day 21, and from day 0 to day 42, plasma lipid peroxidation reduced by an average of 5.39 units and 9.28 units respectively. Both decreases were statistically significant. Lymphocyte antioxidant count for Glutathione-S-transferase and Superoxide dismutase were found to increase from day 0 to day 21, and from day 0 to day 42. The Catalase values decreased during this period. All the mean differences from baseline were found to be statistically significant.

Safety: The laboratory values of parameters for liver function and kidney function were found to be within admissible limits, with minor changes reported during the course of the study. The variation was within 5% of normal values. Similarly blood lipid profiles remained within the normal range. No adverse events were observed in any of the subjects or reported during the study.

Conclusions: The results of this open prospective clinical study suggest that the composition effectively reduces oxidative stress levels, and is safe for use as an antioxidant nutritional supplement.

## Claims

1. A composition comprising garlic powder and a concentrate of selenium compounds comprising about 10 ppm to about 500 ppm of methylselenic acid, about 10 ppm to about 500 ppm of allyl selenocysteine; about 10 ppm to about 500 ppm of L-selenomethionine; about 10 ppm to about 500 ppm of Se-methyl-L selenocysteine; about 10 ppm to about 500 ppm of N-γ (L-Glutamyl) L-selenomethionine; and about 10 ppm to about 500 ppm of N-γ (L-Glutamyl) Se-methyl-L-selenocysteine.

2. The composition as claimed in claim 1, wherein the garlic powder comprises about 10 ppm to about 500 ppm alliin.

3. The composition as claimed in claim 1, wherein the composition is in powder form and provides about 100 ppm to about 2000 ppm of elemental selenium.

4. The composition as claimed in claim 1, wherein the composition further comprises pharmaceutically acceptable carrier.

5. The composition as claimed in claim 1, wherein the composition is for use in human beings or animals for general health maintenance, wherein the composition is for oral administation once a day.

6. The composition as claimed in claim 1, wherein the composition is compressed into a form selected from a group comprising tablet, hard or soft gelatin capsule and as a food product.

## Patentansprüche

1. Zusammensetzung, die Knoblauchpulver und ein Konzentrat aus Selenverbindungen umfasst, das ca. 10 ppm bis ca.500 ppm Methylselensäure, ca. 10 ppm bis ca. 500 ppm Allylselenocystein; ca. 10 ppm bis ca. 500 ppm L-Selenomethionin; ca. 10 ppm bis ca. 500 ppm Se-Methyl-L-Selenocystein; ca. 10 ppm bis ca. 500 ppm N-γ (L-Glutamyl) L-Selenomethionin; und ca. 10 ppm bis ca. 500 ppm N-γ (L-Glutamyl) Se-Methyl-L-Selenocystein umfasst.

2. Zusammensetzung nach Anspruch 1, wobei das Knoblauchpulver ca. 10 ppm bis ca. 500 ppm Alliin umfasst.

3. Zusammensetzung nach Anspruch 1, wobei die Zusammensetzung in Pulverform vorliegt und ca. 100 ppm bis ca. 2000 ppm elementares Selen bereitstellt.

4. Zusammensetzung nach Anspruch 1, wobei die Zusammensetzung darüber hinaus einen pharmazeutisch unbedenklichen Träger umfasst.

5. Zusammensetzung nach Anspruch 1, wobei die Zusammensetzung zur Verwendung bei Menschen oder Tieren zur allgemeinen Gesunderhaltung bestimmt ist, wobei die Zusammensetzung zur oralen Verabreichung einmal täglich bestimmt ist.

6. Zusammensetzung nach Anspruch 1, wobei die Zusammensetzung zu einer Form, die aus einer Gruppe ausgewählt ist, die eine Tablette, eine Hart- oder Weichgelkapsel umfasst, und als Nahrungsmittel gepresst ist.

## Revendications

1. Composition comprenant de la poudre d'ail et un concentré de composés de sélénium comprenant environ 10 ppm à environ 500 ppm d'acide méthylsélénique, environ 10 ppm à environ 500 ppm de sélénocystéine allylique ; environ 10 ppm à environ 500 ppm de L-sélénométhionine ; environ 10 ppm à environ 500 ppm de Se-méthyle-L-sélénocystéine ; environ 10 ppm à environ 500 ppm de N-γ (L-glutamyle) L-sélénométhionine ; et environ 10 ppm à environ 500 ppm de N-γ (L-glutamyle) Se-méthyle-L-sélénocystéine.

2. La composition selon la revendication 1, où la poudre d'ail comprend environ 10 ppm à environ 500 ppm d'alliine.

3. La composition selon la revendication 1, où la composition est sous forme de poudre et contient environ 100 ppm à environ 2000 ppm de sélénium élémentaire.

4. La composition selon la revendication 1, où la composition comprend en outre un entraîneur pharmaceutiquement acceptable.

5. La composition selon la revendication 1, où la composition est destinée à être utilisée chez des êtres humains ou des animaux pour le maintien général de la santé, la composition étant prévue pour l'administration orale une fois par jour.

6. La composition selon la revendication 1, où la composition est comprimée dans une forme choisie dans le groupe comprenant le comprimé, la gélule dure ou molle et en tant qu'un produit alimentaire.
